Europäisches Patentamt

European Patent Office

Office européen des brevets

(19)

(11) Publication number: **0 197 558**
A2

(12) # EUROPEAN PATENT APPLICATION

(21) Application number: 86104873.4

(22) Date of filing: 09.04.86

(51) Int. Cl.⁴: **C 12 N 15/00**
C 12 P 21/02, C 07 K 7/26
C 12 N 1/20

(30) Priority: 11.04.85 GB 8509289

(43) Date of publication of application:
15.10.86 Bulletin 86/42

(84) Designated Contracting States:
BE CH DE FR GB IT LI NL SE

(71) Applicant: Fujisawa Pharmaceutical Co., Ltd.
3, Doshomachi 4-chome Higashi-ku
Osaka-shi, Osaka 541(JP)

(72) Inventor: Ueda, Ikuo
No. 2-11-95, Uenohigashi
Toyonaka-shi Osaka-fu, 560(JP)

(72) Inventor: Niwa, Mineo
No. 7-15, Kirinokuchi Kamiuenocho
Mukoo-shi Kyoto-fu, 617(JP)

(72) Inventor: Saitoh, Yoshimasa
No. 5-14-10 Higashitokiwadai Toyono-cho
Toyono-gun Osaka-fu, 563-01(JP)

(72) Inventor: Satoh, Susumu
No. 7-9-4, Higashitokiwadai Toyono-cho
Toyono-gun Osaka-fu, 563-01(JP)

(72) Inventor: Yamada, Hisashi
No. 779-1, Hokuto 416 Hirano Kamishinzaike
Kawanishi-shi, 666-01(JP)

(74) Representative: Türk, Dietmar, Dr. rer. nat. et al,
Türk, Gille + Hrabal Patentanwälte Bruckner Strasse 20
D-4000 Düsseldorf 13(DE)

(54) Process for production of somatostatin.

(57) Process for preparing somatostatin comprising culturing a microorganism containing therein in recombinant plasmid including a trp promoter, a structural gene coding for the amino acid sequence of a fused protein comprising an additional protein and somatostatin, and one or more termination codon (s), wherein the additional protein is protein peptide LH joined to a peptide derived from a linker and includes a selective cleavage site of methionine adjacent to the N-terminal amino acid sequence of somatostatin, and the recited elements are sequenced in order as mentioned above, and subjecting said fused protein to an elimination reaction of said additional protein. The invention also covers a recombinant plasmid, a microorganism and a fused protein useful in such process.

EP 0 197 558 A2

0197558

## PROCESS FOR PRODUCTION OF SOMATOSTATIN

This invention relates to a process for a microbial production of somatostatin, to a fused protein comprising somatostatin and additional protein, to a recombinant plasmid including a trp promoter and a structural gene coding for the fused protein and to a host microorganism containing the recombinant plasmid.

Somatostatin, which is an object polypeptide having an amino acid sequence as shown below, is known to be useful as a medicament for the treatment of peptic ulcer. Somatostatin is also known to inhibit the secretion of a number of hormones including growth hormone, glucagon and insulin, and accordingly is expected to be useful as a medicament for the treatment of acromegaly and insulin-dependent diabetes.

Somatostatin : Ala-Gly-Cys-Lys-Asn-Phe-Phe-Trp-Lys-
Thr-Phe-Thr-Ser-Cys

While the recombinant DNA technique has been applied to produce useful polypeptides including somatostatin, there is a continuing need for a production of the object polypeptide in high yield.

In fact, US Patent No. 4,425,437 discloses a process for producing somatostatin, in which somatostatin can be produced by cleaving a fused protein of somatostatin and ß-galactosidase which is produced by a recombinant DNA technique.

According to the invention, somatostatin is produced in high yield by employing a new recombinant plasmid characterized in that it includes trp promoter, a structural gene coding for the amino acid sequence of a fused protein comprising an additional protein and somatostatin, and one or more termination codon(s), wherein the additional protein is protein peptide LH joined to a peptide derived from a linker and includes a selective cleavage site of methionine adjacent to the N-terminal amino acid sequence of somatostatin and the recited elements are sequenced as mentioned above.

This recombinant plasmid is transformed into a suitable microorganism, the growth and proliferation of which results in the production of a fused protein comprising an additional protein and somatostatin wherein the additional protein is protein peptide LH joined to a peptide derived from a linker and includes a selective cleavage site of methionine adjacent to the N-terminal amino acid sequence of somatostatin. The fused protein thus obtained is converted to the object polypeptide of somatostatin by subjecting the former to an elimination reaction of the additional protein which is removed at a cleavage site of methionine adjacent to the N-terminal amino acid sequence of somatostatin.

The terms, as used hereinabove and below, have the following meaning unless expressly stated to the contrary.

Suitable "trp promoter gene" may include the gene as derived from the promoter-operator region of Escherichia coli (hereinafter referred to as E. coli), the DNA sequence of which is known [G.N. Bennett et al., J. Mol. Biol. 121, 113 (1978)] and represented in Fig. 1, and the one including at least "promoter region" and "SD sequence (also known as ribosome binding site)".

Preferable example of "trp promoter gene" may include the gene of the promoter-operator region from residues -79 to 22 together with oligonucleotides, which are attached at each of 5' and 3'-ends of said gene to create appropriate cohesive termini for restriction endonucleases such as EcoRI, EcoRI*, HindIII, BamHI or the like.

Most preferable, "trp promoter gene" is the gene of the promoter-operator region from residue -79 to 22, to which EcoRI* site is attached at 5'-end and EcoRI site is attached at 3'-end, and the DNA sequence of "trp promoter gene" is the one as shown in Fig. 2.

"Fused protein" is the one comprising an additional protein and somatostatin is produced by the expression of the structural gene coding for the amino acid sequence of the fused protein in a host microorganism.

Suitable "additional protein", which is easily removed from the fused protein to release somatostatin, may be protein peptide LH joined to a peptide derived from a linker, and may be a selective cleavage site of methionine adjacent to the N-terminal amino acid sequence of somatostatin, greatly enhancing a stability and easiness of the purification of somatostatin.

The amino acid sequence of protein peptide LH is shown in Fig. 3, and preferable example of the DNA sequence of the gene coding for protein peptide LH is also shown therein.

Thus, between protein peptide LH and somatostatin in the fused protein, there exists a peptide derived from a linker, which is introduced so as to facilitate insertion of a structural gene coding for protein peptide LH and somatostatin into a appropriate site on a plasmid.

Suitable "linker oligonucleotide" may include the one having 10-30 base pairs and cohesive termini for the restriction endonucleases such as EcoRI, HindIII, BamHI or the like.

Preferable example of the DNA sequence of the linker is shown in Fig. 4.

Preferable example of the DNA sequence of the gene coding for somatostatin is shown in Fig. 5.

Preferable example of the amino acid sequence of the fused protein is shown in Fig. 6.

Suitable "termination codon(s)" may include TAG, TAA and TGA, which lie downstream the DNA sequence of somatostatin as shown in Fig. 5.

Suitable "plasmid" may include the one derived from microorganisms as conventionally used, such as pBR322, pBR325, pBR328 and the like.

Suitable "microorganism" which is transformed with the recombinant plasmid may include bacteria such as E. coli and the like.

The present invention are explained in more detail by referring to the attached Figures 1 - 12 as follows.

(1) DNA sequence of the elements as included in the plasmid and amino acid sequence of the peptide as expressed are shown in Figures 1 to 6.

Fig. 1     Schematic structure of the gene of trp promoter-operator region of E. coli:

Base-pairs are numbered from the trp m-RNA transcription initiation site designated +1. Restriction endonuclease cleavage sites and the region for "promoter region" and "SD sequence" are as shown.

Fig. 2     DNA sequence of the most preferable trp promoter of the present invention:

Fourteen oligonucleotides are labelled A through N. Base-pairs are numbered as mentioned above.

Fig. 3     Amino acid sequence of the protein peptide LH:

The DNA sequence of the gene coding for the protein peptide LH is also indicated as a preferable example.

Fig. 4     DNA sequence of the most preferable linker:

The corresponding amino acid sequence is also indicated.

Fig. 5    Schematic structure of the gene coding for somatostatin:

Eight oligonucleotides are labelled SA through SH. "Stop" means the termination codons.

Fig. 6    Amino acid sequence of the fused protein:

(2) Methods of synthesis and cloning of the genes of each element are shown in Figures 7 and 8.

Fig. 7    Flow chart for construction of the trp promoter gene joined to somatostatin gene:

The oligonucleotides are annealed and ligated in a series of steps, in order to minimize the possibilities for undesirable interactions as shown in Fig. 7.

An oligonucleotide is illustrated with ●— (● means 5'-phosphorylated end). Ligation is conducted in the presence of T4 DNA ligase.

The oligonucleotides B, C, D, E and F are annealed and ligated to form Block I'. The other Blocks II' - IV' are also obtained by ligation of G through SG as shown.

The oligonucleotide A is ligated with Block I' to give Block V'; and the oligonucleotide SH is ligated with Block IV' to give Block VI'.

Thus obtained Block II', III', V' and VI' are again treated with T4 DNA ligase and the resulting product is digested with EcoRI and BamHI to give the trp promoter gene joined to somatostatin gene.

Fig. 8    Flow chart for construction of plasmid pTrpEB7:

The synthetic trp promoter gene joined to somatostatin gene is inserted into the pBR322 plasmid, previously treated with EcoRI and BamHI restriction endonucleases. The resulting plasmid is transformed into a suitable microorganism such as E. coli HB101. Selection of the transformants for resistance to antibiotics results in the isolation of the plasmid carrying the trp promoter gene joined to somatostatin gene, which is designated as pTrpEB7.

(3)   Methods of synthesis and cloning of a gene fragment containing protein peptide LH gene are shown in Figures 9 and 10.

Fig. 9     Flow chart for construction of a gene fragment containing protein peptide LH gene:

The process for construction is designed to form a gene fragment containing protein peptide LH gene between EcoRI and HindIII restriction sites in the resulting gene.

The explanation as given for Fig. 7 can be referred to.

Fig. 10    Flow chart for molecular cloning of the gene fragment containing protein peptide LH gene:

The gene fragment containing protein peptide LH gene with EcoRI and BamHI termini is inserted into pBR322 plasmid, previously treated with the EcoRI and BamHI restriction endonucleases.

The resulting plasmid is transformed into a suitable microorganism such as E. coli HB101. Selection of the transformants for resistance to antibiotics result in the isolation of the plasmid carrying the gene fragment containing protein peptide LH gene, which is designated as pLH107.

The gene containing the protein peptide LH gene is obtained by the digestion of pLH107 with the EcoRI and BamHI restriction endonucleases.

(4)   Processes for formation of somatostatin gene and construction of plasmid pLHtrp carrying the trp promoter gene and the gene fragment containing protein LH gene are shown in Figure 11.

Fig. 11   Flow chart for construction of somatostatin gene and plasmid pLHtrp:

1)   Somatostatin gene is obtained by the digestion of pTrpEB7 with the EcoRI and BamHI restriction endonucleases.

2)   The plasmid pTrpEB7 is treated with the EcoRI and BamHI restriction endonucleases and the largest fragment containing the trp promoter gene is ligated with the gene fragment containing protein peptide LH gene to give the plasmid pLHtrp.

5)   Processes for construction of plasmid pLHSStrp, which is an object recombinant plasmid of the present invention, are shown in Figure 12.

Fig 12   Flow chart for construction of recombinant plasmid pLHSStrp:

Somatostatin gene with EcoRI and BamHI termini is attached to the linker oligonucleotides to form the gene including somatostatin gene with HindIII and BamHI termini for the insertion into a site of the same restriction specificity on a plasmid.

The plasmid pLHtrp is treated with the HindIII and BamHI restriction endonucleases. The largest fragment containing the trp promoter gene and protein peptide LH gene is ligated with somatostatin gene attached to the linker oligonucleotide having HindIII and BamHI termini to form the plasmid pLHSStrp.

(6)  Process for transforming a microorganism with the recombinant plasmid, expressing the structural gene and producing somatostatin are explained as follows.

For expression of somatostatin gene, thus  . obtained plasmid including the trp promoter gene, protein peptide LH gene and somatostatin gene is transformed into a host microorganism, and then the host microorganism including th recombinant plasmid is cultured in nutrient medium containing sources of assimilable carbon and nitrogen under aerobic conditions (e.g. shaking culture, submerged culture, etc.).

The fermentation is usually conducted at a temperature between about 20°C and 42°C, preferable 35-38°C, for a period of several hours to 50 hours.

Thus produced fused protein comprising additional protein and somatostatin can be recovered from the cultured medium by conventional means which are commonly used for the recovery of other known biologically active substances.

Thus obtained fused protein can be converted to somatostatin by elimination reaction of the additional protein.

This elimination reaction can be conducted in accordance with a conventional method used in the field of peptide chemistry such as method of using cyanogen bromide or the like.

This reaction is usually carried out under mild conditions in a conventional solvent which does not adversely affect the reaction.

The reaction temperature is not critical and the reaction is usually carried out from cooling to warming.

Abbreviations used herein are as follows

A and Ap as used in oligonucleotide sequence are adenine,

T and Tp as used in oligonucleotide sequence are thymine,

G and Gp as used in oligonucleotide sequence are guanine, and

C and Cp as used in oligonucleotide sequence are cytosine.

$A^{Bz}po$ is adenine wherein an amino and hydroxy groups are protected by benzoyl and p-chlorophenyl, respectively,

Tpo is thymine wherein an hydroxy group is protected by p-chlorophenyl,

$G^{iB}po$ is guanine wherein an amino and hydroxy groups are protected by isobutyryl and p-chlorophenyl, respectively,

$C^{Bz}po$ is cytosine wherein an amino and hydroxy groups are protected by benzoyl and p-chlorophenyl, respectively,

$^{Ac}Upo$ is uracyl wherein an hydroxy group is protected by acetyl, and

DMTr: dimethoxytrityl.

The following Preparations and Examples are given for the purpose of illustrating the present invention.

## Preparation 1

Synthesis of HOGpGpTpTpGpTpApApGpApApCpTpTpCpTOH (SB)

which corresponds to the oligonucleotide as labelled SB in Fig. 7.

(1) Synthesis of DMTrOTpoTpoC$^{Bz}$poTpo$^{Ac}$Upo-cellulose:

i) Preparation of HOTpo$^{Ac}$Upo-cellulose:

To a suspension of DMTrOTpo$^{Ac}$Upo-cellulose (250.0 mg, 26.1 µmole*) (prepared by R. Crea's method[1]) in methanol/chloroform (1:9 v/v, 5.0 ml). Trichloroacetic acid/chloroform (2:8 w/v , 5.0 ml) was added under ice cooling, and the mixture was stirred at 0°C for 10 minutes. After being washed with chloroform (2 ml) and methanol (6.0 ml), successively, on the filter, the cellulose adduct (HOTpo$^{Ac}$Upo-cellulose) was dried, water being separated as the pyridine (2 ml) azeotrope.

* This value was calculated by monitoring the absorbance of a washing solution with chloroform at 507 nm.

1) R. Crea et al, Nucleic Acid Research 8, 2331(1980)

ii) Preparation of DMTrOTpoTpoC$^{Bz}$po$^-$:

DMTrOTpoTpoC$^{Bz}$po-CE (223 mg) was treated with triethylamine-acetonitrile (1:1 v/v, 5 ml) at room temperature for 1 hour. The phosphodiester trimer (DMTrOTpoTpoC$^{Bz}$po$^-$) so obtained was dried, water being separated as the pyridine azeotrope (0.5 ml, 2 x 1 ml).

iii) Coupling:

The trimer (DMTrOTpoTpoC$^{Bz}$po$^-$) was mixed with the cellulose adduct (HOTpo$^{Ac}$Upo-cellulose) in a 10 ml round-bottom flask. The mixture was dried, water being separated as the pyridine azeotrope (2 x 1 ml) and finally resuspended in anhydrous pyridine (1 ml). Mesitylen sulfonyl nitrotriazolide (193 mg, 652.5 μmole) was added to the suspension and the mixture was stirred at room temperature for 1 hour. And then pyridine was added to the reaction vessel and cellulose adduct was recovered by centrifugation (3,000 rpm, 2 minutes).

iv) Acetylation of unreacted 5'-hydroxy groups:

The cellulose adduct obtained as above was suspended in a solution of pyridine-acetic anhydride (10:1 v/v, 5.5 ml) and stirred at room temperature for 30 minutes. The cellulose-product (258.4 mg) was obtained by repeated centrifugation (3,000 rpm, 2 minutes) in pyridine (5 ml), washing with methanol (15 ml) and drying in vacuo at room temperature for 30 minutes. The cellulose adduct (DMTrOTpoTpoC$^{Bz}$poTpo$^{Ac}$Upo-cellulose) can use for the next coupling step.

(2) Synthesis of $DMTrOA^{Bz}poA^{Bz}poC^{Bz}poTpoTpoC^{Bz}poTpo^{Ac}Upo$-cellulose:

$DMTrOA^{Bz}poA^{Bz}poC^{Bz}poTpoTpoC^{Bz}poTpo^{Ac}Upo$-cellulose was synthesized from $DMTrOTpoTpoC^{Bz}poTpo^{Ac}Upo$-cellulose (258.4 mg) and $DMTrOA^{Bz}poA^{Bz}poC^{Bz}po$-CE (252 mg) according to similar conditions as above.

(3) Synthesis of $DMTrOA^{Bz}poA^{Bz}poG^{iB}poA^{Bz}poA^{Bz}poC^{Bz}poTpoTpoC^{Bz}poTpo^{Ac}Upo$-Cellulose:

$DMTrOA^{Bz}poA^{Bz}poG^{iB}poA^{Bz}poA^{Bz}poC^{Bz}poTpoTpoC^{Bz}poTpo^{Ac}Upo$-cellulose (256.4 mg) was synthesized from $DMTrOA^{Bz}poA^{Bz}poC^{Bz}poTpoTpoC^{Bz}poTpo^{Ac}Upo$-cellulose (260.4 mg) and $DMTrOA^{Bz}poA^{Bz}poG^{iB}po$-CE (253 mg) according to similar conditions.

(4) Synthesis of $DMTrOTpoG^{iB}poTpoA^{Bz}poA^{Bz}poG^{iB}poA^{Bz}poA^{Bz}poC^{Bz}poTpoTpoC^{Bz}poTpo^{Ac}Upo$-cellulose:

$DMTrOTpoG^{iB}poTpoA^{Bz}poA^{Bz}poG^{iB}poA^{Bz}poA^{Bz}poC^{Bz}poTpoTpoC^{Bz}poTpo^{Ac}Upo$-cellulose (234.0 mg) was synthesized from $DMTrOA^{Bz}poA^{Bz}poG^{iB}poA^{Bz}poA^{Bz}poC^{Bz}poTpoTpoC^{Bz}poTpo^{Ac}Upo$-cellulose (256.4 mg) and $DMTrOTpoG^{iB}poTpo$-CE (224 mg) according to similar conditions.

(5) Synthesis of $DMTrOG^{iB}poG^{iB}poTpoTpoG^{iB}poTpoA^{Bz}poA^{Bz}poG^{iB}poA^{Bz}poA^{Bz}poC^{Bz}poTpoTpoC^{Bz}poTpo^{Ac}Upo$-cellulose:

$DMTrOG^{iB}poG^{iB}poTpoTpoG^{iB}poTpoA^{Bz}poA^{Bz}poG^{iB}po$-

$A^{Bz}poA^{Bz}poC^{Bz}poTpoTpoC^{Bz}poTpo^{Ac}Upo$-cellulose (224.0 mg) was synthesized from DMTrOTpoG$^{iB}poTpoA^{Bz}poA^{Bz}po$-G$^{iB}poA^{Bz}poA^{Bz}poC^{Bz}poTpoTpoC^{Bz}poTpo^{Ac}Upo$-Cellulose (234.0 mg) and DMTrOG$^{iB}poG^{iB}poTpo$-CE (236 mg) under the similar conditions. At this final process, unreacted 5'-hydroxy group was not necessary to protect with an acetyl group.

(6) Synthesis of HOGpGpTpTpGpTpApApGpApApCpTpTpCpTOH:

DMTrOG$^{iB}poG^{iB}poTpoTpoG^{iB}poTpoA^{Bz}poA^{Bz}poG^{iB}po$-A$^{Bz}poA^{Bz}poC^{Bz}poTpoTpoC^{Bz}poTpo^{Ac}Upo$-cellulose (224.0 mg) was treated with 0.5 M N,N,N',N'-tetramethyl-guanidinium pyridine 2-aldoximate (in dioxane-$H_2O$ (1:1 v/v, 1 ml)) at 20°C for 20 hours in a sealed tube. To the reaction mixture 28% (w/w) aqueous ammonia (16 ml) was added, and the mixture was heated at 60°C for 5 hours. The solid cellulose was removed by filtration and washed with water (10 ml). The filtrate and washed solution were evaporated to dryness, and the residue was treated with 80% aqueous acetic acid (150 ml) at room temperature for 15 minutes. After removal of the solvents, the residue was dissolved in 0.1 M triethylammonium carbonate buffer (pH 7.5, 25 ml) was washed with diethylether (3 x 25 ml). Aqueous layer was evaporated to dryness and the residue was dissolved in 0.1 M triethylammonium carbonate buffer (pH 7.5, 2 ml) to yield crude HOGpGpTpTpGpTpApApGpApApCpTpTpCpTOH in the solution.

(7) Purification of HOGpGpTpTpGpTpApApGpApApCpTpTpCpTOH

i) First purification of the crude product was performed by column chromatography on Biogel P2 (column: 24 x 2.6 cm, inner diameter, trademark: made by Biolad). The fractions corresponding to the first eluted peak (50 mM ammonium acetate, 0.1 mM EDTA, 1 ml/minute) were collected and freeze-dried to give the first purified product.

ii) The second purification of the first purified product was performed by high pressure liquid column chromatograpy (hereinafter referred to as HPLC) on CDR-10 (column: 25 cm x 4.6 mm, inner diameter, trademark: made by Mitsubishi Chemical Industries Ltd.) using a linear gradient of 1M ammonium acetate-10% (v/v) aqueous ethanol to 4.5 M ammonium acetate-10% (v/v) aqueous ethanol (80 minutes, 1 ml/minute, 60°C) to give the second purified product.

iii) The third purification of the second purified product was performed by reverse phase HPLC [column: Rp-18-5µ(x77), 15 cm x 4mm, inner diameter, trademark: made by Merck] using a linear gradient of 0.1 M ammonium acetate to 0.1 M ammonium acetate 15% (v/v) in aqueous acetonitrile (40 minutes, 1.5 ml/minute, room temperature) to give the final purified product (HOGpGpTpTpGpTpApApGpApApCpTpTpCpTOH).

(8) Analysis of oligonucleotide:
     (HOGpGpTpTpGpTpApApGpApApCpTpTpCpTOH)

i) Digestion by phosphodiesterase:

     The mixture of
HOGpGpTpTpGpTpApApGpApApCpTpTpCpTOH (30 µg, 130 µl),
0.2 M magnesium chloride (45 µl), 0.2 M
TRIS-hydrochloric acid (pH 8.5) (45 µl) and 0.1 mM
EDTA in an aqueous solution (170 µl) was treated with

phosphodiesterase (from <u>Crotalus</u> <u>durissus</u>) (60 µg, 60 µl purchased from Boehringer Mannheim) at 37°C for 30 minutes, and then heated at 100°C for 2 minutes.

ii) Analysis by HPLC:

The oligonucleotide in the reaction mixture was analyzed by HPLC on CDR-10 using a linear gradient of water to 2.0 M ammonium acetate (pH 3.4) (40 minutes, 1.5 ml/minute, 60°C). From each peak area observed, its nucleotide composition was determined comparing with area of a standard sample.

Calcd: $pC_{OH}$ 2,000, $pA_{OH}$ 4,000, $pT_{OH}$ 6,000, $pG_{OH}$ 3,000
Observed: $pC_{OH}$ 2,030, $pA_{OH}$ 4,257, $pT_{OH}$ 5,843, $pG_{OH}$ 2,871

<u>Preparation 2</u>

Synthesis of oligonucleotides (SA, SC, SD, SE, SF, SG, and SH which correspond to the symbols of each oligonucleotide in Fig. 7):

Following oligonucleotides were prepared by a similar manner to that of Preparation 1.

(1) HOApApTpTpCpApTpGpGpCpTOH (SA)

(2) HOTpTpTpGpGpApApGpApCpTpTpTOH (SC)

(3) HOCpApCpTpTpCpGpTpGpTpTpGpApTpApGOH (SD)

(4) HOTpTpApCpApApCpCpApGpCpCpApTpGOH (SE)

(5) HOCpCpApApApApGpApApGpTpTpCOH (SF)

(6)  HOCpGpApApGpTpGpApApApGpTpCpTpTOH (SG)


(7)  HOGpApTpCpCpTpApTpCpApApCpAOH (SH)


Preparation 3

Synthesis of oligonucleotides (A, B, C, D, E, F, G, H, I, J, K, L, M and N which correspond to the symbols of each oligonucleotide in Fig. 7):

Following oligonucleotide were prepared by a similar manner to that of Preparation 1.

(1)  HOApApTpTpTpGpCpCpGpApCpAOH  (A)

(2)  HOCpGpTpTpApTpGpApTpGpTpCpGpGpCpAOH  (B)

(3)  HOTpCpApTpApApCpGpGpTpTpCpTpGpGpCOH  (C)

(4)  HOGpApApTpApTpTpTpGpCpCpApGpApApCOH  (D)

(5)  HOApApApTpApTpTpCpTpGpApApApTpGpAOH  (E)

(6)  HOTpCpApApCpApGpCpTpCpApTpTpTpCpAOH  (F)

(7)  HOGpCpTpGpTpTpGpApCpApApTpTpApApTOH  (G)

(8)  HOGpTpTpCpGpApTpGpApTpTpApApTpTpGOH  (H)

(9)  HOCpApTpCpGpApApCpTpApGpTpTpApApCOH  (I)

(10)  HOGpCpGpTpApCpTpApGpTpTpApApCpTpAOH  (J)

(11)  HOTpApGpTpApCpGpCpApApGpTpTpCpApCOH  (K)

(12)  HOCpTpTpTpTpTpApCpGpTpGpApApCpTpTOH (L)

(13)  HOGpTpApApApApApGpGpGpTpApTpCpGOH (M)

(14)  HOApApTpTpCpGpApTpApCpCOH (N)

## Preparation 4

Following linker oligonucleotide (m1 and m2) were prepared by a similar manner to that of Preparation 1.

(1)  HOApGpCpTpTpGpApApGpTpApApApApCpApTpGOH (m1)

(2)  HOApApTpTpCpApTpGpTpTpTpTpApCpTpTpCpAOH (m2)

## Preparation 5

Preparation of trp promoter gene joined to somatostatin gene (see Fig. 7):

Aliquots of each oligonucleotides (A through N, SA through SH) (0.4 nM) were phosphorylated with 4 units of T4 polynucleotide kinase (made by BRL) in 100 µl of a solution containing 74 mM TRIS-hydrochloric acid (pH 7.6), 10 mM dithiothreitol, 1.6 mM mercaptoethanol, 10 mM magnesium chloride and 0.5 mM ATP for 20 minutes at 37°C. After the reaction was completed, the enzyme in the reaction mixture was deactivated by incubation at 100°C for 5 minutes. Ligation of the phosphorylated oligonucleotides was carried out as shown in Fig. 7 to give firstly fragment four blocks and ultimately the trp promoter gene joined to somatostatin gene for cloning. Ligations were carried out with T4 DNA ligase (7 units) in a solution containing 100 mM ATP (0. 5 µl) for 23 hours at 4°C (standard conditions). The ligation products of oligonucleotides in each step were identified by staining with ethidium bromide following electroelution on a 2-16% gradient polyacrylamide gel electrophoreisis (hereinafter referred to as PAGE) in tris-EDTA buffer. The last ligation product was digested with EcoRI and BamHI and purified by preparative 7.5% PAGE to give the somatostatin gene joined to trp promoter gene (163 bp).

Preparation 6

Molecular cloning of somatostatin gene joined to trp promoter gene (see Fig. 8):

The synthetic gene was ligated with EcoRI, BamHI fragment of pBR322 (commercially available) in the presence of T4 DNA ligase and then E. coli HB101 (ATCC 33694) was transformed with the ligation product according to Kushner's protocol [T.Maniatis, E. F. Fritsch, J. Sambrook: Molecular Cloning p. 252 (1982)]. The plasmid obtained from the transformant of $^R$Amp and $^S$Tet was digested with HpaI to confirm a band (4.1 kbp), and then digested with BamHI to confirm a band of 90 bp on PAGE. Moreover, the somatostatin gene 56 bp by EcoRI-BamHI digestion was confirmed by the comparison with size marker on PAGE. This plasmid was named pTrpEB7 and used for a construction of plasmid pLHtrp.

Preparation 7

Synthesis of oligonucleotides (a1, a2, a3, a4, a5, a6, b1, b2, b3, b4, b5, b6, c1, c2, c3, c4, c5, c6, d1,

d2, d3, d4, d5, d6, e1, e2, e3, e4, e5, 11, 12 and 13 which correspond to the symbols of each oligonucleotide in Fig. 9):

Following oligonucleotides were prepared by a similar manner to that of SB described in Preparation 1.

(1) HOApApTpTpCpApTpGpTpGpTpTOH (a1)

(2) HOApCpTpGpCpCpApGpGpApCpCpCpApTOH (a2)

(3) HOApTpGpTpApApApApGpApApGpCpApGOH (a3)

(4) HOTpGpGpCpApGpTpApApCpApCpApTpGOH (a4)

(5) HOTpTpTpApCpApTpApTpGpGpGpTpCpCOH (a5)

(6) HOApApGpGpTpTpTpTpCpTpGpCpTpTpCpTOH (a6)

(7) HOApApApApCpCpTpTpApApGpApApApApTpAOH (b1)

(8) HOCpTpTpTpApApTpGpCpApGpGpTpCpAOH (b2)

(9) HOTpTpCpApGpApTpGpTpApGpCpGpGpAOH (b3)

(10) HOApTpTpApApApGpTpApTpTpTpCpTpTOH (b4)

(11) HOApTpCpTpGpApApTpGpApCpCpTpGpCOH (b5)

(12) HOTpTpCpCpApTpTpApTpCpCpGpCpTpApCOH (b6)

(13) HOTpApApTpGpGpApApCpTpCpTpTpTpTpCOH (c1)

(14) HOTpTpApGpGpCpApTpTpTpTpGpApApGOH (c2)

- 21 -

0197558

(15)  HOApApTpTpGpGpApApApGpApGpGpApGOH  (c3)

(16)  HOTpGpCpCpTpApApGpApApApApGpApGOH  (c4)

(17)  HOTpCpCpApApTpTpCpTpTpCpApApApAOH  (c5)

(18)  HOCpTpGpTpCpApCpTpCpTpCpCpTpCpTpTOH  (c6)

(19)  HOApGpTpGpApCpApGpApApApApApTpAOH  (d1)

(20)  HOApTpGpCpApGpApGpCpCpApApApTpTOH  (d2)

(21)  HOGpTpCpTpCpCpTpTpTpTpApCpTpTOH  (d3)

(22)  HOCpTpCpTpGpCpApTpTpApTpTpTpTOH  (d4)

(23)  HOApGpGpApGpApCpApApTpTpTpGpGOH  (d5)

(24)  HOApApApGpCpTpTpGpApApGpTpApApAOH  (d6)

(25)  HOCpApApGpCpTpTpTpTpCpApApApApAOH  (e1)

(26)  HOCpTpTpTpApApGpGpApTpGpApCpCpAOH  (e2)

(27)  HOGpApGpCpApTpCpCpApApApApGpApGOH  (e3)

(28)  HOCpCpTpTpApApApGpTpTpTpTpGpAOH  (e4)

(29)  HOGpGpApTpGpCpTpCpTpGpGpTpCpApTOH  (e5)

(30)  HOTpGpTpGpTpApApTpGpApTpApGOH  (11)

(31)  HOTpApCpApCpApCpTpCpTpTpTpTOH  (12)

(32)  HOGpApTpCpCpTpApTpCpApTOH  (13)

Preparation 8

Preparation of a gene fragment containing protein peptide LH gene (see Fig. 9):

Aliquots of each oligonucleotides (a2-12) (0.4 nM) were phosphorylated with 2.5 units of T4 polynucleotide kinase in 40 µl of a solution containing 50 mM TRIS-hydrochloric acid (pH 7.6), 20 mM dithiothreitol, 50 µg/ml bovine serum albumin, 1 mM spermidine, 10 mM magnesium chloride and 2 mM ATP for 3 hours at 37°C. After the reaction was completed, the enzyme in the reaction mixture was deactivated by incubation at 100°C for 5 minutes. Ligation of the phosphorylated oligonucleotides and two oligonucleotides (a1 and 13) was carried out as shown in Fig. 9 to give firstly fragment six blocks and ultimately protein peptide LH gene (236 bp) for cloning. Ligation was carried out with T4 DNA ligase (5 units) in a solution containing 50 mM ATP (1 µl) for 5 hours at 16°C. The ligation products of oligonucleotides in each step were identified by staining with ethidium bromide following electroelution on a 2-16% gradient PAGE in TRIS-EDTA buffer.

Preparation 9

Molecular cloning of the gene fragment containing protein peptide LH gene (see Fig. 10):

Plasmid pBR322 was digested with BamHI and EcoRI restriction endonucleases. Reaction was terminated by heating at 65°C for 5 minutes and the fragments separated by electrophoresis on a 0.5% agarose gel.

The 3985 bp large fragment from pBR322 was recovered and ligated with the gene fragment containing protein peptide LH gene (236 bp) synthesized as above in the presence of T4 DNA ligase for 18 hours at 12°C. The ligated mixture was transformed into E. coli HB101 by Kushner's method and ampicillin resistant transformants were selected on the plate containing tetracycline (25 µg/ml). The plasmid obtained from a transformant resistant to ampicillin and sensitive to tetracycline was digested with EcoRI and BamHI and the fragments were compared with appropriate size markers on an agarose gel electrophoresis. The expected 236 bp gene fragment containing protein peptide LH fragment was generated. This plasmid was named pLH107 and was used for the construction of plasmid pLHtrp.

Preparation 10

Construction of plasmid pLHtrp (see Fig. 11):

pTrpEB7 prepared in Preparation 6 was digested with EcoRI and BamHI to give a large fragment (4.1 kbp) by preparative agarose gel electrophoresis. This fragment was ligated with the gene fragment containing protein peptide LH gene prepared from a plasmid pLH107 by EcoRI and BamHI digestion. The ligated mixture was transformed into E. coli HB101 to give ampicillin resistant and tetracycline sensitive transformants. The plasmid (pLHtrp) obtained from the transformant was digested with EcoRI and BamHI to confirm the gene fragment containing protein peptide LH gene (236 bp) on 7.5% PAGE.

Example 1

Construction of somatostatin recombinant plasmid pLHSStrp (see Fig. 12):

Plasmid pTrpEB7 was digested with EcoRI and BamHI to give somatostatin gene (56 bp). On the other hand, oligonucleotide (m2) as prepared in Preparation 4 (2) was phosphorylated with T4 polynucleotide kinase as described in Preparation 5. The phosphorylated oligonucleotide, oligonucleotide (m1) as prepared in Preparation 4 (1) and somatostatin gene (56 bp) were mixed and treated with T4 ligase in a solution containing 100mM ATP for 20 hours at 4°C. The ligation mixture was digested with BamHI and then purified by preparative PAGE to give somatostatin gene with linker (74 bp). The gene (74 bp) was ligated with the fragment obtained from pLHtrp by HindIII-BamHI digestion, and then the ligation mixture was transformed into E. coli HB101. The E. coli HB101 containing plasmid pLHSStrp was named E. coli SS-1. The plasmid (pLHSStrp) obtained from the transformant was digested with EcoRI and BamHI (198, 56 bp), HindIII and BamHI (74 bp), HpaI-BamHI (288 bp) to confirm trp promoter gene, protein peptide LH and somatostatin gene on 7.5% PAGE.

## Example 2

Expression of a gene coding for somatostatin fused with protein peptide LH in E. coli SS-1:

An overnight culture of E. coli SS-1 (which is E. coli HB101 containing plasmid pLHSStrp) in L broth containing 50µg/ml ampicillin was diluted 1:20 in M9 medium containing 0.2% glucose, 0.5% casamino acid (acid-hydrolyzed casein), 50 µg/ml vitamin B1 and 25

µg/ml ampicillin. ß-Indole acrylic acid was added to a final concentration of 10µg/ml when $A_{600}$ was 0.5. Then the cells were incubated for 3 hours and collected by centrifugation to give wet cell paste. (8 krpm, 4°C, 10 minutes).

Example 3

Isolation and purification of somatostatin:

Wet cell paste prepared in Example 2 was suspended in 70% formic acid containing cyanogen bromide (10 mg/ml) and stirred at room temperature overnight. The solvent was removed by lyophilization, the residue was suspended in 8M urea buffer (8M urea, 0.1M TRIS-hydrochloric acid (pH 8.0), (0.1M dithiothreitol) and adjusted with TRIS at pH 8.0. After centrifugation of the solution, the supernatant was purified by gel filtration on HPLC (column: TSK 3000SW, trademark: made by Toyo Soda Manufacturing Co. Ltd.). The fraction eluted with 8M urea buffer was purified by reverse phase HPLC (column: Beckman Ultrapore RPSC, trademark: made by Beckman, linear gradient from 10% to 60% acetonitrile in 0.01M trifluoroacetic acid) to obtain the purified somatostatin. N-terminal sequence [Ala(1) - Phe(7)] of the somatostatin was determined by amino acid sequence analysis. By this procedure about 1 mg of purified somatostatin was obtained from 1 litter of culture liquid.

Example 4

1) Isolation and purification of fused somatostatin:

Wet cell paste prepared by the method of Example 2 (6g) was suspended in 10mM PBS-EDTA (pH 8.0) buffer (15ml) and cells were lysed by sonication. The cells debris was pelleted by centrifugation at 18,000 rpm for 30 minutes. The pellet was dissolved in 10ml of 0.1M TRIS-hydrochloric acid (pH 8.0), 6M guanidine HCl, 10m M EDTA and 0.1M dithiothreitol and stirred at 4°C overnight, then centrifuged at 4°C (15,000 rpm, 30 minutes). The supernatant was collected and applied to a gel filtration by HPLC (TSK 3000SW column) equilibrated with 6M guanidine HCl, 0.1M TRIS-hydrochloric acid (pH 8.0), 10mM EDTA, and 10mM dithiotheitol. Elution was carried out at 4°C with elution buffer, at a flow rate of 0.7 ml/minute. The fractions having a peak at retention time of 30 minutes were collected and were dialyzed against 1M acetic acid aqueous solution. The fraction dialyzed was lyophilized to give fused somatostatin. The fused somatostatin shows a band at the position of molecular weight 9,500 on 15% sodium dodecyl sulfate-polyacrylamide gel electrophoreisis.

2) Elimination of protein peptide LH from fused somatostatin with cyanogen bromide:

The fused somatostatin obtained above was dissolved in 1ml of 80% formic acid. Cyanogen bromide (3mg) was added and the mixture was allowed to stand at room temperature overnight. The solvent was removed by lyophylization, and the residue was dissolved in 1ml of 6M guanidine HCl, 0.1M TRIS-hydrochloric acid (pH 8.0), 10mM EDTA and 0.1M dithiothreitol.

HPLC:

The solution obtained above was applied.

column:    Beckman Ultrapore RpSC (made by
           Beckman)

flow rate: 1 ml/minute

elution:   linear gradient from 10% to 60%
           acetonitrile in 0.01M trifluoroacetic
           acid over 50 minutes.

The chromatography was repeated and fractions having a peak with a retention time of 17.23 minutes were collected. The pooled fraction was lyophilized to give somatostatin.

Fig. 1    Schematic structure of the gene of
          the trp promoter-operator region of E. coli

Fig. 2    DNA sequence of the most preferable
          trp promoter gene of the invention

(EcoR I*)
    -80                        -60

    ←———A———→ ←———C———→ ←———E———→
    AATTTGCCGACATCATAACGGTTCTGGCAAATATTCTGAAATGA
      ACGGCTGTAGTATTGCCAAGACCGTTTATAAGACTTTACTCGACAACT
    ←———B———→ ←———D———→ ←———F———→

    -40                    -20    (HpaI)         1                    20    (EcoRI)

    ←———G———→ ←———I———→ ←———K———→ ←———M———→
    GCTGTTGACAATTAATCATCGAACTAGTTAACTAGTACGCAAGTTCACGTAAAAAGGGTATCG
      GTTAATTAGTAGCTTGATCAATTGATCATGCGTTCAAGTGCATTTTTCCCATAGCTTAA
    ←———H———→ ←———J———→ ←———L———→ ←—N—→

0197558

Fig. 3    Amino acid sequence of
the protein peptide LH


```
                          1
            (EcoRI)Met Cys Tyr Cys Gln Asp Pro Tyr
             5'-AATTC-ATG-TGT-TAC-TGC-CAG-GAC-CCA-TAT-
             3'-G-TAC-ACA-ATG-ACG-GTC-CTG-GGT-ATA-


       10                                              20
Val Lys Glu Ala Glu Asn Leu Lys Lys Tyr Phe Asn Ala Gly
GTA-AAA-GAA-GCA-GAA-AAC-CTT-AAG-AAA-TAC-TTT-AAT-GCA-GGT-
CAT-TTT-CTT-CGT-CTT-TTG-GAA-TTC-TTT-ATG-AAA-TTA-CGT-CCA-


                          30
His Ser Asp Val Ala Asp Asn Gly Thr Leu Phe Leu Gly Ile
CAT-TCA-GAT-GTA-GCG-GAT-AAT-GGA-ACT-CTT-TTC-TTA-GGC-ATT-
GTA-AGT-CTA-CAT-CGC-CTA-TTA-CCT-TGA-GAA-AAG-AAT-CCG-TAA-


                          40
Leu Lys Asn Trp Lys Glu Glu Ser Asp Arg Lys Ile Met Gln
TTG-AAG-AAT-TGG-AAA-GAG-GAG-AGT-GAC-AGA-AAA-ATA-ATG-CAG-
AAC-TTC-TTA-ACC-TTT-CTC-CTC-TCA-CTG-TCT-TTT-TAT-TAC-GTC-


  50                                  (Hind III)
Ser Gln Ile Val Ser Phe Tyr Phe
AGC-CAA-ATT-GTC-TCC-TTT-TAC-TTC-A
TCG-GTT-TAA-CAG-AGG-AAA-ATG-AAG-TTC-GA
```

Fig. 4    DNA sequence of the most preferable linker


(HindIII)    LeuGluValLysHis  (EcoRI)
        AGCTTGAAGTAAAACATG
            ACTTCATTTTGTACTTAA

Fig. 5    Schematic structure of the gene coding for somatostatin

(EcoRI)    AlaGlyCysLysAsnPhePheTrpLysThrPheThrSerCys stop (Bam HI)

    ◄———SA———►◄———SB———►◄———SC———►◄———SD———►
AATTCATGGCTGGTTGTAAGAACTTCTTTTGGAAGACTTTCACTTCGTGTTGATAG
    GTACCGACCAACATTCTTGAAGAAAACCTTCTGAAAGTGAAGCACAACTATCCTAG
    ◄———SE———►◄———SF———►◄———SG———►◄———SH———►

- 32 -

0197558

Fig. 6    Amino acid sequence of the fused protein


1
Met- Cys –Tyr– C ys–Gln–Asp–Pro –Tyr—

10                                                              20
Val–Lys–Glu–Ala–Glu–Asn–Leu–Lys–Lys–Tyr–Phe–Asn–Ala–Gly—

30
His–Ser–Asp–Val–Ala–Asp–Asn–Gly–Thr–Leu–Phe–Leu–Gly–Ile—

40
Leu–Lys–Asn–Trp–Lys–Glu–Glu–Ser–Asp–Arg–Lys–Ile–Met–Gln—

50                                                              60
Ser– Gln–Ile–Val–Ser–Phe–Tyr–Phe–Lys–Leu–Glu–Val–Lys–His—

70
Glu–Phe–Met–Ala–Gly–Cys–Lys–Asn–Phe–Phe–Trp–Lys–Thr–Phe—

80
Thr–Ser–Cys

0197558

Fig. 7    Construction of the trp promoter gene
         jointed to somatostatin gene

trp promoter gene jointed to
somatostatin gene

Fig. 8     Construction of plasmid pTrpEB7

Fig. 9    Construction of a gene fragment
containing protein peptide LH gene

gene fragment
containing protein LH gene

0197558

**Fig. 10**    Molecular cloning of a gene fragment
containing promoter peptide LH gene

Fig. 11    Construction of somatostatin gene and
           plasmid pLHtrp

PTrpEB7

cleavage by
EcoRI and BamHI

somatostatin gene

gene fragment
containing protein
peptide LH gene

pLHtrp

E. coli HB101 containing pLHtrp

pLHtrp

Fig. 12    Construction of recombinant plasmid pLHSStrp

somatostatin gene with linker

somatostatin gene

pLHSStrp

E. coli HB101 containing pLHSStrp (E. coli SS-1)

pLHSStrp

What we claim is:

1. A process for preparing somatostatin, characterized in that it comprises

(a) culturing a microorganism containing therein a recombinant plasmid including a trp promoter, a structural gene coding for the amino acid sequence of a fused protein comprising an additional protein and somatostatin, and one or more termination codon(s), wherein the additional protein is protein peptide LH joined to a peptide derived from a linker and includes a selective cleavage site of methionine adjacent to the N-terminal amino acid sequence of somatostatin, and the recited elements are sequenced in order as mentioned above,

(b) isolating said fused protein from culture broth, and

(c) subjecting said fused protein to an elimination reaction of said additional protein to give somatostatin.

2. A recombinant plasmid including a trp promoter, a structural gene coding for the amino acid sequence of a fused protein comprising an additional protein and somatostatin, and one or more termination codon(s), wherein the additional protein is protein peptide LH joined to a peptide derived from a linker and includes a selective cleavage site of methionine adjacent to the N-terminal amino acid sequence of somatostatin, and the recited elements are sequenced in order as mentioned above.

3. A microorganism containing a recombinant plasmid including a trp promoter, a structural gene coding for the amino acid sequence of a fused protein comprising an additional protein and somatostatin, and one or more termination codon(s), wherein the additional protein is protein peptide LH joined to a peptide derived from a linker and includes a selective cleavage site of methionine adjacent to the N-terminal amino acid sequence of somatostatin, and the recited elements are sequenced in order as mentioned above.

4. A fused protein comprising additional protein and somatostatin wherein additional protein is protein peptide LH jointed to a peptide derived from a linker.

5. A fused protein of Claim 4, which has a following amino acid sequence:

```
        1                                    10
Met-Cys-Tyr-Cys-Gln-Asp-Pro-Tyr-Val-Lys-Glu-Ala-

                        20
Glu-Asn-Leu-Lys-Lys-Tyr-Phe-Asn-Ala-Gly-His-Ser-Asp-

                   30
Val-Ala-Asp-Asn-Gly-Thr-Leu-Phe-Leu-Gly-Ile-Leu-Lys-
```

40                                                    50
Asn-Trp-Lys-Glu-Glu-Ser-Asp-Arg-Lys-Ile-Met-Gln-Ser-

                                                60
Gln-Ile-Val-Ser-Phe-Tyr-Phe-Lys-Leu-Glu-Val-Lys-His-

                        70
Glu-Phe-Met-Ala-Gly-Cys-Lys-Asn-Phe-Phe-Trp-Lys-Thr-

                80
Phe-Thr-Ser-Cys